# FASCICULE DE BREVET EUROPEEN

(11) **EP 0 533 827 B1**
(45) Date de publication et mention de la délivrance du brevet: **24.08.1994**
(21) Numéro de dépôt: 91912243.2
(22) Date de dépôt: 13.06.1991
(51) Int. Cl.: C07D 413/06, A61K 31/42

(54) **NOUVEAUX DERIVES DE L'OXAZOLE, LEUR PREPARATION ET LES COMPOSITIONS PHARMACEUTIQUES QUI LES CONTIENNENT**
OXAZOLEDERIVATE, IHRE HERSTELLUNG UND DIESE ENTHALTENDE PHARMAZEUTISCHEN ZUBEREITUNGEN
NOVEL OXAZOLE DERIVATIVES, A PREPARATION METHOD THEREFOR AND PHARMACEUTICAL COMPOSITIONS CONTAINING SAME

(30) Priorité: 14.06.1990 FR 9007388
(43) Date de publication de la demande: 31.03.1993
(73) Titulaire: LABORATOIRE ROGER BELLON, 92200 Neuilly-sur-Seine (FR)
(72) Inventeur: BARREAU, Michel, F-91230 Montgeron (FR); KRYVENKO, Michel, F-75116 Paris (FR); LAVERGNE, Marc, Pierre, F-94250 Mandres-les-Roses (FR); TECHER, Auguste, F-77219 Avon (FR)
(74) Mandataire: Savina, Jacques
(86) Numéro de dépôt international: FR9100473
(87) Numéro de publication internationale: WO9119714

(56) Documents cités:
- GB-A- 1 206 403
- GB-A- 1 373 352
- GB-A- 1 381 860

## Description

La présente invention concerne des dérivés de l'oxazole de formule générale : 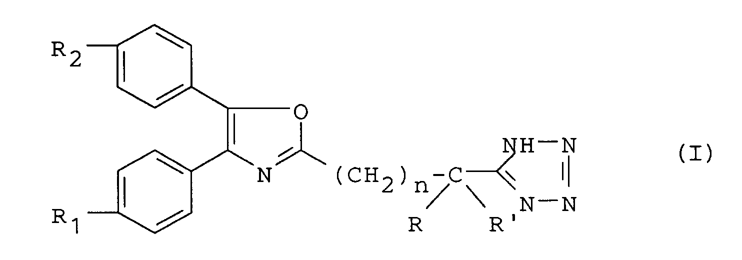
dans laquelle :
R et R' sont identiques ou différents et représentent un atome d'hydrogène ou un radical alcoyle contenant 1 ou 2 atomes de carbone, R₁ et R₂ sont identiques ou différents et représentent des atomes d'hydrogène ou d'halogène, ou des radicaux alcoyloxy dont la partie alcoyle contient 1 à 4 atomes de carbone en chaîne droite ou ramifiée, et
n égale 3 à 6,
ainsi que leurs sels, leurs isomères lorsqu'ils existent et les compositions pharmaceutiques qui les contiennent.

Dans le brevet britannique GB 1 381 860 ont été décrits des dérivés de tétrazoles de structure : 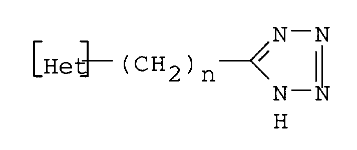
dans laquelle [Het] est un hétérocycle à 5 chainons de formule générale: 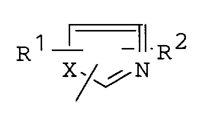
pouvant être O ou S et R¹ et R² étant des groupes aryle substitués ou non,
et , n égale 0, 1 ou 2.

Ces dérivés manifestent une activité anti-inflammatoire.

Dans la demande de brevet FR 1 584 222 ont été décrits les dérivés de l'oxazole de formule générale : 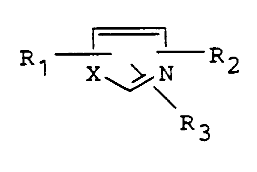
dans laquelle R₁ et R₂ sont identiques ou différents et représentent des radicaux phényle éventuellement substitués par halogène, alcoyle, alcoyloxy, nitro ou amino, X est un atome d'oxygène ou de soufre et R₃ est un radical acide aliphatique contenant 2 à 6 atomes de carbone.

Ces produits présentent une activité anti-inflammatoire.

Dans la demande de brevet DE 2 129 012 ont été décrits les dérivés d'azole de formule générale : 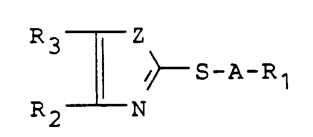
dans laquelle R₁ est un groupe carboxy, R₂ et R₃ sont des groupes aryles éventuellement substitués, A est CₙH₂ₙ, et Z est un atome de soufre ou d'oxygène.

Ces produits sont utiles comme anti-inflammatoires.

Il a été trouvé maintenant, que les dérivés de l'oxazole selon l'invention qui possèdent des chaînes longues, séparant les hétérocycles, manifestent une action antagoniste des effets du leucotriène B₄, que ne possédaient pas ou pratiquement pas les produits de l'art antérieur.

Le leucotriène B₄ est un puissant médiateur de l'inflammation qui est formé à la suite de la biotransformation de l'acide arachidonique par la voie de la 5-lipoxygénase. Il contribue en particulier à des phénomènes comme le chimiotactisme, l'activation cellulaire, l'exocytose d'enzymes et participe également aux dérèglements immunologiques et tissulaires. Les produits selon l'invention sont donc particulièrement intéressants dans le traitement des maladies où ce médiateur joue un rôle, notamment dans le traitement des maladies inflammatoires, pour lesquelles les produits de l'art antérieur étaient inefficaces.

Dans la formule générale (I), lorsque les radicaux R₁ et/ou R₂ représentent des atomes d'halogène, ils peuvent être choisis parmi les atomes de fluor, de chlore ou de brome. De préférence ils représentent l'atome de chlore. Le symbole n est compris entre 3 et 6, mais de préférence on choisira n égal à 4 ou 5.

Selon l'invention les dérivés de l'oxazole peuvent être préparés par action d'un azoture alcalin ou de l'azoture de trin.butyl étain sur un nitrile de formule générale : 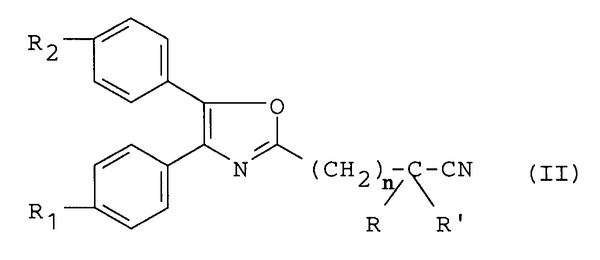
dans laquelle R, R', R₁, R₂ et n sont définis comme précédemment.

Lorsque l'on fait réagir un azoture alcalin, on opère avantageusement par action de l'azoture de sodium. La réaction s'effectue en présence de chlorure d'ammonium ou du chlorhydrate d'une base organique azotée (triéthylamine, di-n.butylamine par exemple), dans un solvant organique tel qu'un amide (diméthylformamide, N,N-diméthylacétamide, N-méthylpyrrolidone par exemple), à une température comprise entre 80 et 150°C, de préférence entre 80 et 110°C. Lorsque l'on opère par action de l'azoture de tri-n.butyl étain, la réaction s'effectue dans le diméthoxy-1,2 éthane, à la température définie ci-dessus.

Le nitrile de formule générale (II) pour lequel R ou R' est un atome d'hydrogène peut être préparé par hydrolyse du cyanoester de formule générale : 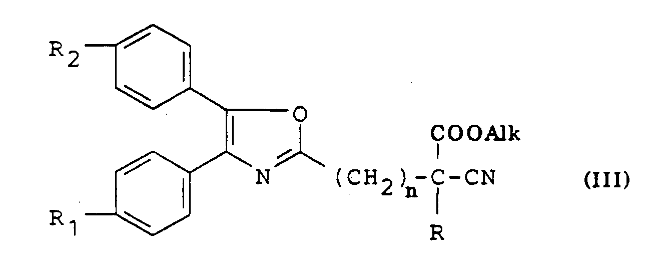
dans laquelle R, R₁, R₂ et n sont définis comme précédemment, et Alk représente un radical alcoyle droit ou ramifié contenant 1 à 4 atomes de carbone, suivie de la décarboxylation de l'acide obtenu.

L'hydrolyse de l'ester peut être réalisée en milieu acide ou basique. Lorsque l'on opère en milieu acide, la réaction s'effectue par exemple par action de l'acide p.toluènesulfonique ou de l'acide formique à une température comprise entre 100 et 130°C. Lorsque l'on opère en milieu basique, on opère avantageusement par action d'une base alcaline comme la soude ou la potasse, en solution hydroalcoolique (par exemple en milieu méthanol-eau, éthanol-eau, ou méthoxyéthanol-eau), à une température comprise entre 5 et 50°C.

La décarboxylation de l'acide s'effectue par chauffage à une température comprise entre 100 et 200°C.

Le cyanoester de formule générale (III) peut être préparé par action d'un cyanoacétate de formule générale : 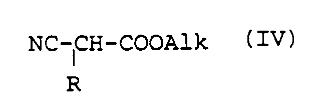
dans laquelle Alk et R sont définis comme ci-dessus, sur le dérivé bromé de formule générale : 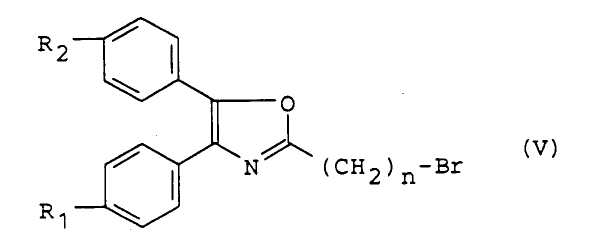
dans laquelle R₁, R₂ et n sont définis comme précédemment.

La réaction est catalysée par l'iodure de tétrabutylammonium; elle s'effectue en présence d'un accepteur d'acide par exemple un carbonate alcalin (carbonate de potassium), dans un solvant
aprotique polaire. On opère avantageusement dans le diméthylformamide à une température comprise entre 50 et 110°C.

Il n'est pas indispensable de purifier le produit obtenu pour le mettre en oeuvre dans la réaction suivante.

Le dérivé bromé de formule générale (V) peut être obtenu par cyclisation du cétoamide de formule générale : 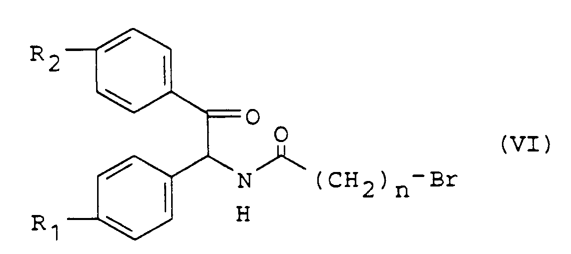
dans laquelle R₁, R₂ et n sont définis comme précédemment.

On opère en présence d'un agent de déshydratation comme par exemple l'oxychlorure de phosphore, le chlorure de thionyle, l'acide chlorosulfonique ou en présence d'un arylsulfochlorure (chlorure de benzènesulfonyle, chlorure de tosyle) dans la pyridine. La réaction s'effectue avec ou sans solvant à une température comprise entre 5 et 150°C. Lorsque l'on opère dans un solvant, celui-ci est avantageusement choisi parmi le cyclohexane, les solvants aromatiques (toluène) ou les solvants chlorés (chlorure de méthylène, dichloro-1,2 éthane).

Le dérivé bromé de formule générale (VI) peut être obtenu par action du chlorure d'acide de formule générale :

Cl-CO-(CH₂)ₙ-Br (VII)

dans laquelle n est défini comme précédemment, sur le chlorhydrate de l'aminocétone de formule générale : 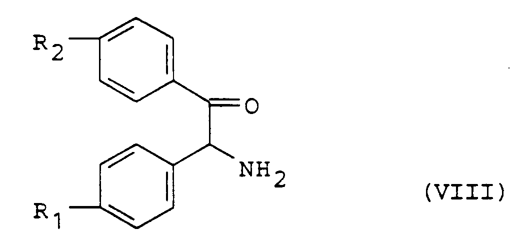
dans laquelle R₁ et R₂ sont définis comme précédemment.

La réaction s'effectue généralement en présence d'un excès du chlorure d'acide de formule générale (VII), et d'une base organique azotée comme la pyridine ou une amine tertiaire (triéthylamine, N-méthylmorpholine ou N,N-diméthylaniline par exemple), ou d'un carbonate alcalin. On opère avantageusement dans un solvant chloré (chloroforme, chlorure de méthylène), dans un éther (éther éthylique, tétrahydrofuranne, dioxanne, diméthoxy-1,2 éthane) ou dans un hydrocarbure aliphatique ou aromatique, à une température comprise entre 5 et 120°C.

Le chlorhydrate de l'aminocétone de formule générale (VIII) peut être préparé selon les méthodes décrites par :
- G. Drefahl et Coll., Ann. Chem., 589, 82 (1954) et J.Prakt.Chem., 32, 307 (1966);
- M.J. Hatch et Coll., J. Am. Chem. Soc., 75, 38 (1953);
- H.O. House et Coll., J. Org. Chem., 28, 307 (1963);
ou comme décrit ci-après dans les exemples.

Le nitrile de formule générale (II) dans laquelle R et R' représentent des atomes d'hydrogène peut être également obtenu par action d'un cyanure alcalin sur un dérivé bromé de formule générale : 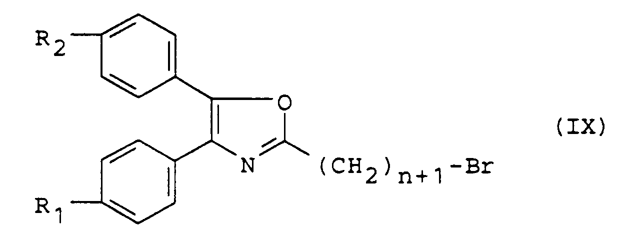
dans laquelle R₁, R₂ et n sont définis comme précédemment.

La réaction s'effectue généralement en milieu hydro-alcoolique à la température de reflux du mélange réactionnel. On opère avantageusement par action du cyanure de potassium, en milieu hydro-éthanolique.

Le dérivé bromé de formule générale (IX) peut être préparé par analogie avec la préparation du dérivé bromé de formule générale (V).

Le nitrile de formule générale (II) dans laquelle R et R' représentent simultanément des radicaux alcoyle, peut être préparé par action d'un nitrile de formule générale : 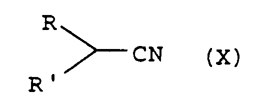
dans laquelle R et R' sont définis comme ci-dessus, sur un dérivé bromé de formule générale (V).

La réaction s'effectue avantageusement en présence de diisopropylamidure de lithium dans un solvant organique ou un mélange de solvants comme par exemple le tétrahydrofuranne et le toluène, à une température voisine de -70°C.

Le nitrile de formule générale (II) pour lequel R est hydrogène et R' est alcoyle peut également être préparé par cyclisation du nitrile de formule générale : 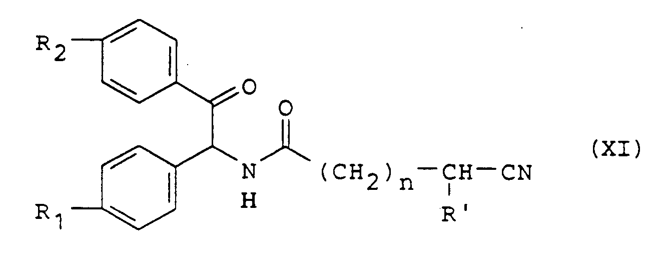
dans laquelle R₁, R₂ et n sont définis comme précédemment et R' est défini comme ci-dessus.

La réaction s'effectue dans les conditions décrites précédemment pour la cyclisation d'un dérivé de formule générale (VI).

Le nitrile de formule générale (XI) peut être obtenu par action d'un chlorure d'acide de formule générale : 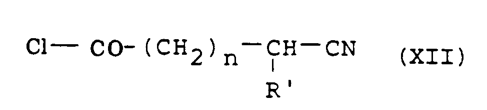
dans laquelle n et R' sont définis comme précédemment pour la formule générale (XI), sur le chlorhydrate de l'aminocétone de formule générale (VIII).

La réaction s'effectue dans des conditions analogues aux conditions décrites pour la réaction du chlorure d'acide de formule générale (VII) avec le chlorhydrate de l'aminocétone de formule générale (VIII).

Le chlorure d'acide de formule générale (XII) peut être préparé comme décrit ci-après dans les exemples, ou par analogie avec cette méthode.

Les produits selon l'invention peuvent être purifiés par cristallisation ou par chromatographie.

Le cas échéant les isomères des produits selon l'invention peuvent être séparés selon les méthodes habituelles qui n'altèrent pas le reste de la molécule. Par exemple ils peuvent être séparés par chromatographie sur une colonne chirale.

Les produits selon la présente invention peuvent être transformés en sels métalliques selon les méthodes connues en soi. Ces sels peuvent être obtenus par action d'une base métallique forte sur un produit selon l'invention dans un solvant approprié. Le sel formé précipite après concentration éventuelle de sa solution, il est séparé par filtration, décantation ou lyophilisation.

Comme exemples de sels pharmaceutiquement acceptables, peuvent être cités les sels avec les métaux alcalins (sodium, potassium, lithium).

Les nouveaux dérivés de l'oxazole de formule générale (I) et leurs sels sont particulièrement intéressants dans le domaine de l'inflammation où intervient le leucotriène B₄, notamment dans le domaine ostéoarticulaire. Du fait de leur affinité pour les récépteurs à leucotriène B₄, ils interfèrent avec cet agoniste en bloquant son action au niveau du récepteur.

Leur affinité pour les récepteurs à leucotriène B₄ a pu être mise en évidence en mesurant leur effet vis à vis du binding à leucotriène B₄ tritié sur des membranes de rate de cobaye, selon une méthode inspirée de la méthode de J.B. Cheng, J. of Pharmacology and Experimental Therapeutics, 236, 126 (1986). Dans cette technique les produits selon l'invention se sont montrés actifs à des concentrations comprises entre 5 et 500 nM (CI₅₀).

Les produits sélectionnés ont également montré qu'ils étaient antagonistes du leucotriène B₄ en s'opposant in vivo à l'afflux cellulaire induit par injection de LTB₄ dans le péritoine de la souris, selon une technique inspirée de la méthode décrite par D.E.Griswold et coll., Inflammation, 13(6), 727 (1989). Dans cette technique, les produits se sont montrés actifs à des doses comprises entre 20 et 100 mg/kg par voie orale.

De plus les produits selon l'invention présentent l'intérêt d'être très faiblement toxiques. Leur toxicité (DL₅₀) chez la souris est comprise entre 200 mg/kg et des valeurs supérieures à 1 g/kg par voie orale.

D'un intérêt particulier sont les dérivés de l'oxazole pour lesquels :
R et R' identiques ou différents sont des atomes d'hydrogène ou des radicaux méthyle,
R₁ et R₂ identiques ou différents représentent des atomes d'hydrogène ou de chlore ou des radicaux méthoxy et
n égale 4 ou 5, et parmi ces produits plus spécialement les dérivés de l'oxazole suivants :
- {[bis (méthoxy-4 phényl)-4,5 oxazolyl-2]-6 hexyl}-5 tétrazole;
- {[bis(méthoxy-4 phényl)-4,5 oxazolyl-2]-7 heptyl-2}-5 1H-tétrazole;
- {[bis(méthoxy-4 phényl)-4,5 oxazolyl-2]-7 méthyl-2 heptyl-2}-5 1H-tétrazole;
- {[bis(méthoxy-4 phényl)-4,5 oxazolyl-2]-5 pentyl}-5 tétrazole;
- {[(chloro-4 phényl)-4 (méthoxy-4 phényl)-5 oxazolyl-2]-6 hexyl}-5 tétrazole
Les exemples suivants donnés à titre non limitatif illustrent la présente invention.

### EXEMPLE 1

On chauffe sous agitation, pendant 50 heures à 105°C, 42 g de [bis(méthoxy-4 phényl)-4,5 oxazolyl-2]-7 heptanenitrile en solution dans 850 cm³ de diméthylformamide avec 21 g d'azoture de sodium et 17,3 g de chlorure d'ammonium.

Le mélange est alors ramené à la température ambiante, additionné d'une nouvelle quantité d'azoture de sodium (21 g) et de chlorure d'ammonium (17,3 g) puis agité à nouveau à 105°C pendant 60 heures. Après refroidissement, les produits insolubles sont séparés par filtration, la solution diluée par 2550 cm³ d'eau puis amenée à pH 3 par l'acide chlorhydrique 2N et extraite avec 3 fois 300 cm³ d'acétate d'éthyle. Les solutions organiques réunies sont lavées par décantation, d'abord par 3 fois 100 cm³ d'eau puis par 2 fois 100 cm³ d'une solution aqueuse saturée en chlorure de sodium. Après séchage sur sulfate de magnésium le solvant est évaporé sous pression réduite (5,2 kPa). Le résidu de l'évaporation est chromatographié sur une colonne de 5,8 cm de diamètre intérieur, contenant 420 g de silice (50 à 200 µ). On élue, en recueillant des fractions de 200 cm³, par 4,2 litres d'oxyde de diisopropyle puis par 2 litres d'un mélange d'oxyde de diisopropyle- acétate-d'éthyle (70-30 en volume) et enfin par 2,2 litres d'oxyde de diisopropyle-acétate d'éthyle (50-50). Les résidus solides, résultant de l'évaporation des fractions du dernier mélange éluant sont réunis et recristallisés dans 130 cm³ d'acétate d'éthyle. On obtient 20,4 g (43,7 %) de {[bis (méthoxy-4 phényl)-4,5 oxazolyl-2]-6 hexyl}-5 tétrazole, sous la forme de cristaux blancs fondant à 124°C.

Le [bis(méthoxy-4 phényl)-4,5 oxazolyl-2]-7 heptanenitrile peut être préparé comme suit :
On chauffe à 180°C, 67,5 g d'acide [bis(méthoxy-4 phényl)-4,5 oxazolyl-2]-7 cyano-2 heptanoïque, jusqu'à cessation du dégagement de gaz carbonique (7 heures). Après refroidissment, on dissout l'huile résiduelle dans 400 cm³ d'éther éthylique. On lave la solution éthérée avec 3 fois 50 cm³ d'une solution aqueuse saturée de bicarbonate de sodium, 100 cm³ d'eau, sèche sur sulfate de magnésium, filtre et élimine le solvant. On chromatogaphie le résidu de l'évaporation sur une colonne de 5,8 cm³ de diamètre, contenant 360 g de gel de silice (50 à 200 µ). On élue, en recueillant des fractions de 250 cm³, par 3,5 litres d'un mélange d'oxyde de diisopropyle-acétate d'éthyle (70-30 en volume). On concentre à sec les fractions comprises entre 0,5 et 3,5 litres. On obtient ainsi 52,3 g (75,9 %) de {[bis (méthoxy-4 phényl)-4,5 oxazolyl-2]-7 heptanenitrile, sous la forme d'une huile directement utilisée dans la réaction précédente.

L'acide[bis(méthoxy-4 phényl)-4,5 oxazolyl-2]-7 cyano-2 heptanoïque peut être préparé de la manière suivante : A une solution de 84,8 g de bis (méthoxy-4 phényl)-1,2 (bromo-6 hexanamido)-2 éthanone dans 320 cm³ de toluène, on ajoute, sous agitation 53 cm³ d'oxychlorure de phosphore. On chauffe le mélange 4 heures à 80°C, puis concentre à sec sous pression réduite (5,2 kPa). On ajoute au résidu 250 cm³ d'eau glacée et extrait avec du dichlorométhane (3 fois 150 cm³). Les extraits organiques réunis sont lavés à l'eau (2 fois 100 cm³), une solution aqueuse saturée de bicarbonate de sodium (2 fois 100 cm³) et séchés sur sulfate de magnésium. Après filtration, on concentre à sec sous pression réduite (0,2 kPa) et obtient 78,1 g (95,9 %) d'une huile visqueuse.

On dissout celle-ci dans 75 cm³ de diméthylformamide et ajoute la solution au mélange de 103 g de cyanacétate d'éthyle, 6,7 g d'iodure de tétrabutylammonium et 25 g de carbonate de potassium dans 550 cm³ de diméthylformamide. On agite la suspension à 60°C pendant 12 heures. Après refroidissment, on ajoute 2,5 litres d'eau, extrait avec de l'oxyde de diéthyle (3 fois 500 cm³). On lave les extraits éthérés réunis avec 3 fois 75 cm³ d'eau, sèche sur sulfate de magnésium, filtre et concentre à sec sous pression réduite (5,2 kPa). On chromatographie le résidu sur une colonne de 5,8 cm de diamètre, contenant 300 g de gel de silice. On élue, en recueillant des fractions de 200 cm³, par 2 litres d'un mélange à parties égales d'oxyde de diisopropyle et d'hexane, et on élimine ces fractions, puis par 3,5 litres d'oxyde de diisopropyle pur qui sont concentrés à sec sous pression réduite (0,2 kPa). On obtient 59,3 g de résidu visqueux. Celui-ci est dissous dans 490 cm³ d'éthanol. On y ajoute 25,4 cm³ de soude 10N et agite la solution 3 heures à 20°C. On évapore les solvants, ajoute au résidu une solution de 22 cm³ d'acide chlorhydrique (d = 1,18) dans 110 cm³ d'eau. On essore le précipité, lave à l'eau et sèche sous le vide phosphorique. On obtient 49,1 g d'acide [bis(méthoxy-4 phényl)-4,5 oxazolyl-2]-7 cyano-2 heptanoïque, sous la forme d'une poudre blanchâtre fondant à 150°C avec effervescence (décarboxylation).

La bis(méthoxy-4 phényl)-1,2 (bromo-6 hexanamido)-2 éthanone peut être préparé de la manière suivante : A une suspension, refroidie à 5°C et agitée, de 64,6 g de chlorhydrate d'amino-2 bis(méthoxy-4 phényl)-1,2 éthanone (obtenue selon G. DREFAHL et M. HARTMAN, Ann. Chem., 1954, 589, p. 82-90) dans 250 cm³ de dichlorométhane contenant 47 g de chlorure de l'acide bromo-6 hexanoïque, on ajoute en 50 minutes une solution de 41,5 g de pyridine dans 50 cm³ de dichlorométhane. On agite le mélange 20 heures à la température ambiante. On ajoute 60 cm³ d'eau, décante la phase organique qui est lavée avec 2 fois 50 cm³ d'acide chlorhydrique normal puis à l'eau (2 fois 30 cm³). On sèche sur sulfate de magnésium et évapore le solvant. Le résidu solide est recristallisé dans un mélange: oxyde de diisopropyle (1300 cm³) et isopropanol (80 cm³). On obtient 84,9 g (90 %) de bis(méthoxy-4 phényl)-1,2 (bromo-6 hexanamido)-2 éthanone fondant à 93°C.

### EXEMPLE 2

On opère selon l'exemple 1. On chauffe 60 heures à 105°C un mélange de 3,4 g de [bis(méthoxy-4 phényl)-4,5 oxazolyl-2]-6 hexanenitrile, 1,45 g d'azoture de sodium et 1,76 g de chlorure d'ammonium dans 70 cm³ de diméthylformamide. Après l'addition de quantités égales aux premières d'azoture de sodium et de chlorure d'ammonium, on porte à nouveau à 105°C pendant 60 heures. A la température ambiante, on dilue avec 210 cm³ d'eau, acidifie avec de l'acide chlorhydrique 2N, extrait à l'acétate d'éthyle (3 fois 50 cm³) et purifie le produit de la réaction comme indiqué à l'exemple 1. On recristallise le solide isolé dans 60 cm³ d'acétate d'éthyle et on obtient 1,7 g (44,8 %) de {[bis(méthoxy-4 phényl)-4,5 oxazolyl-2]-5 pentyl}-5 tétrazole, solide blanc fondant à 156°C.

Le [bis(méthoxy-4 phényl)-4,5 oxazolyl-2]-6 hexanenitrile est préparé de la manière suivante:
On porte, 15 heures au reflux, une solution de 5 g de bis (méthoxy-4 phényl)-4,5 (bromo-5 pentyl)-2 oxazole dans 23 cm³ d'éthanol et 1,1 g de cyanure de potassium dissous dans 9 cm³ d'eau. Après l'évaporation du solvant, on dissout le résidu organominéral dans 40 cm³ d'oxyde de diéthyle et 10 cm³ d'eau, on sépare la phase éthérée et lave celle-ci avec 10 cm³ d'une solution saturée de chlorure de sodium sèche sur sulfate de magnésium, filtre et concentre à sec. Le résidu visqueux (4,4 g) est chromatographié sur une colonne de 2 cm de diamètre utile contenant 80 g de gel de silice (50 à 200 µ). On élue avec 1,2 litres d'oxyde de diisopropyle en recueillant des fractions de 50 cm³. Les fractions comprises entre 0,2 et 1,2 litres sont concentrées à sec. On obtient 3,5 g (80 %) de [bis(méthoxy-4 phényl)-4,5 oxazolyl-2]-6 hexanenitrile sous la forme d'une huile jaune pâle.

### EXEMPLE 3

Selon les conditions (chauffage et temps) définies dans l'exemple 2, on traite 1,5 g de [bis(chloro-4 phényl)-4,5 oxazolyl-2]-7 heptanenitrile dans 30 cm³ de diméthylformamide par 2 fois 0,73 g de l'azoture de sodium et 2 fois 0,60 g de chlorure d'ammonium. On chromatographie le produit brut isolé (1,6 g) sur une colonne de 2 cm de diamètre contenant 20 g de gel de silice (50 à 200 µ). On élue, en recueillant des fractions de 100 cm³, d'abord par 0,9 litres d'oxyde de diisopropyle puis par 0,6 litres d'acétate d'éthyle. On concentre à 30 cm³ les éluats de l'ester acétique, refroidit la solution et obtient 0,8 g (48%) de {[bis(chloro-4 phényl)-4,5 oxazolyl-2]-6 hexyl}-5 tétrazole, sous la forme de cristaux blancs fondant à 148°C.

Le [bis(chloro-4 phényl)-4,5 oxazolyl-2]-7 heptanenitrile peut être préparé de la manière suivante : On dissout 8,8 g de bis(chloro-4 phényl)-4,5 (bromo-5 pentyl)-2 oxazole dans 60 cm³ de diméthylformamide contenant 14,1 g de cyanoacétate de tert.-butyle. On ajoute à la solution 2,8 g de carbonate de potassium et 0,8 g d'iodure de tétrabutylammonium. On agite le mélange 32 heures à 45-50°C. Après refroidissement, on ajoute 200 cm³ d'eau et extrait avec de l'oxyde de diéthyle (3 fois 50 cm³). On lave les extraits organiques à l'eau (3 fois 20 cm³), sèche sur sulfate de magnésium, filtre et concentre à sec. On chromatographie le résidu sur une colonne de 3 cm de diamètre contenant 200 g de gel de silice (50 à 200 µ).

On élue, en recueillant des fractions de 100 cm³, par 2 litres d'un mélange oxyde de diéthyle-hexane (20-80 en volumes). On concentre à sec les fractions comprises entre 0,5 et 2 litres. On obtient 10 g d'une huile jaunâtre.

On dissout celle-ci dans 20 cm³ d'acide formique pur et agite la solution à 20°C pendant 21 heures. On concentre à sec en chauffant à 170°C jusqu'à cessation du dégagement de gaz carbonique. On refroidit, puis ajoute au résidu 20 cm³ d'eau et extrait avec de l'éther éthylique (3 fois 20 cm³). On lave les extraits organiques réunis avec 50 cm³ de bicarbonate de sodium en solution aqueuse saturée, à l'eau (20 cm³), sèche sur sulfate de magnésium, filtre et concentre à sec. On chromatographie le résidu sur une colonne (diamètre 2 cm), contenant 64 g de gel de silice (50 à 200 µ). On élue avec 0,9 litres de chlorure de méthylène en recueillant des fractions de 50 cm³. On concentre à sec les fractions comprises entre 0,4 et 0,9 litres. On obtient 1,6 g (20 %) de [bis(chloro-4 phényl)-4,5 oxazolyl-2]-7 heptanenitrile, sous la forme d'une poudre blanche fondant à 64°C.

On prépare la bis(chloro-4 phényl)-4,5 (bromo-5 pentyl)-2 oxazole de la manière suivante : On agite 3 heures à 80°C, 39,5 g de bis(chloro-4 phényl)-1,2 (bromo-6 hexanamido)-2 éthanone dans 80 cm³ de toluène contenant 40 g d'oxychlorure de phosphore. Après évaporation à sec sous vide, on ajoute 100 cm³ d'eau glacée. On extrait avec de l'oxyde du diéthyle (3 fois 120 cm³) et lave les solutions éthérées, successivement : eau (3 fois 50 cm³) ; bicarbonate de sodium en solution aqueuse saturée (2 fois 100 cm³). On sèche et concentre à sec. On dissout le résidu solide dans 400 cm³ d'hexane, traite au noir animal, filtre sur KIESELGUHR, refroidit le filtrat à 10°C, essore le précipité et sèche. On obtient 33 g (87 %) de bis (chloro-4 phényl)-4,5 (bromo-5 pentyl)-2 oxazole, sous la forme de cristaux blancs fondant à 98°C.

On peut préparer la bis(chloro-4 phényl)-1,2 (bromo-6 hexanamido)-2 éthanone de la manière indiquée pour le cétoamide de l'exemple 1. A partir de 37,1 g de chlorhydrate d'amino-2 bis(chloro-4 phényl)-1,2 éthanone (préparée selon M.J. HATCH et D.J. CRAM, J. Amer.Chem. Soc., 1953, 75, p.38 à 44), 26,2 g du chlorure de l'acide bromo-6 hexanoïque et 140 cm³ de chlorure de méthylène, on ajoute 23,4 g de pyridine dans 30 cm³ de chlorure de méthylène. On opère selon l'exemple 1 et on cristallise le résidu solide des traitements dans 300 cm³ d'oxyde de diisopropyle. On obtient 40,5 g (75 %) de bis (chloro-4 phényl)-1,2 (bromo-6 hexanamido)-2 éthanone, cristaux blancs fondant à 105°C.

### EXEMPLE 4

On opère selon l'exemple 2 en chauffant pendant 48 heures 2,4 g de [bis(chloro-4 phényl)-4,5 oxazolyl-2]-6 hexanenitrile, 1,2 g de l'azoture de sodium et 1 g de chlorure d'ammonium dans 45 cm³ de diméthylformamide. On ajoute une quantité égale à la première d'azoture de sodium et de chlorure d'ammonium et porte à nouveau à 105°C pendant 48 heures. On extrait le produit de la réaction comme dans l'exemple cité ci-dessus et cristallise dans 80 cm³ d'oxyde de diisopropyle. On obtient 1,2 g de {[bis(chloro-4 phényl)-4,5 oxazolyl-2]-5 pentyl}-5 tétrazole, cristaux blancs fondant à 100°C.

On peut préparer le [bis(chloro-4 phényl)-4,5 oxazolyl-2]-6 hexanenitrile, selon l'exemple 2, en chauffant 12 heures au reflux, 4,4 g de bis(chloro-4 phényl)-4,5 (bromo-5 pentyl)-2 oxazole de l'exemple 3 dans 40 cm³ d'éthanol additionnés de 0,9 g de cyanure de potassium dissous dans 8 cm³ d'eau. Après recristallisation dans 50 cm³ d'oxyde de diisopropyle, on obtient 2,5 g (64 %) de [bis(chloro-4 phényl)-4,5 oxazolyl-2]-6 hexanenitrile, cristaux blancs fondant à 108°C.

### EXEMPLE 5

On opère selon l'exemple précédent en chauffant 1 g de [(diphényl)-4,5 oxazolyl-2]-7 heptanenitrile, deux fois 0,9 g d'azoture de sodium et deux fois 0,7 g de chlorure d'ammonium dans 20 cm³ de diméthylformamide. On cristallise le résidu solide issu des extractions dans un mélange d'acétate d'éthyle (5 cm³) et d'oxyde de diisopropyle (15 cm³). On obtient 0,4 g (35 %) de {[diphényl)-4,5 oxazolyl-2]-6 hexyl}-5 tétrazole, sous forme de cristaux blancs fondant à 132°C.

On peut préparer le [(diphényl)-4,5 oxazolyl-2]-7 heptanenitrile, selon l'exemple 3 :
A partir de 15 g de (bromo-6 hexanamido)-2 diphényl-1,2 éthanone, 17,8 g d'oxychlorure de phosphore dans 35 cm³ de toluène (4 heures à 80°C), on obtient 11,5 g d'une huile jaunâtre. 7,4 g de cette huile sont chauffés 26 heures à 50°C, sous agitation, dans 60 cm³ de diméthylformamide contenant 14,1 g de cyanoacétate de tert.-butyle, 0,8 g d'iodure de tétrabutylammonium et 2,8 g de carbonate de potassium. On chromatographie le résidu des extraits organiques de la réaction sur une colonne de diamètre 3 cm contenant 200 g de gel de silice. On élue, en recueillant des fractions de 100 cm³, par 2,5 litres d'un mélange d' oxyde de diisopropyle et d'hexane (vol./vol). On concentre à sec les fractions comprises entre 1,5 et 2,5 litres. On obtient 2,5 g d'une huile jaunâtre.

On dissout cette huile dans 50 cm³ de xylol contenant 0,1 g d'acide p.toluènesulfonique et chauffe 7 heures au reflux. Après évaporation à sec, on ajoute 60 cm³ d'une solution saturée de bicarbonate de sodium et 120 cm³ d'oxyde de diéthyle. On lave la solution éthérée avec 30 cm³ d'eau, sèche sur sulfate de magnésium et concentre à sec. On chromatographie le résidu sur une colonne diamètre 2 cm contenant 50 g de gel de silice (50 à 200 µ). On élue, en recueillant des fractions de 50 cm³, avec 0,4 litre de diisopropyle oxyde puis 0,5 litre d'acétate d'éthyle. On concentre à sec les éluats de l'ester acétique et on obtient 1 g d'une huile épaisse et jaunâtre, directement mise en oeuvre dans l'étape initiale de l'exemple.

On peut préparer la (bromo-6 hexanamide)-2 diphényl-1,2 éthanone, selon l'exemple 1 :
A partir de 12,9 g de chlorhydrate d'amino désoxybenzoïne (préparé selon H.O. HOUSE et W.F. BERKOWITZ, J. Org. Chem., 28, 307 (1963)) et 11,7 g du chlorure de bromo-6 hexanoyle dans 65 cm³ de chlorure de méthylène et 10,3 g de pyridine dans 15 cm³ de chlorure de méthylène, on obtient, après cristallisation dans 100 cm³ d'oxyde de diisopropyle, 15,1 g (74,6 %) de (bromo-6 hexanamido)-2 diphényl-1,2 éthanone, sous la forme de cristaux blancs fondant à 92°C.

### EXEMPLE 6

Selon l'exemple 5, on chauffe 4 g de [(chloro-4 phényl)-4 (méthoxy-4 phényl)-5 oxazolyl-2]-7 heptanenitrile, deux fois 2 g d'azoture de sodium et deux fois 1,7 g de chlorure d'ammonium dans 50 cm³ de diméthylformamide. On cristallise le résidu solide issu des extraits organiques 3 fois 75 cm³ d'oxyde de diéthyle dans 50 cm³ de toluène et on obtient 2,5 g (56 %) de {[(chloro-4 phényl)-4 (méthoxy-4 phényl)-5 oxazolyl-2]-6 hexyl}-5 tétrazole, cristaux blancs fondant à 132°C.

On peut préparer la [(chloro-4 phényl)-4 (méthoxy-4 phényl)-5 oxazolyl-2]-7 heptanenitrile, en trois étapes, selon la séquence des réactions de l'exemple 2.

A une suspension, agitée et refroidie à 5°C., de 6,2 g de chlorhydrate d'amino-2 (chloro-4 phényl)-2 (méthoxy-4 phényl)-1 éthanone (G. DREFAHL, G. HEUBLEIN, K. FRITZSCHE et R. SIEMANN, J. Prakt. Chem, 1966, 32, p. 307 à 310) dans 50 cm³ de dichlorométhane contenant 4,9 g de chlorure de bromo-7 heptanoyle, on ajoute 4 g de pyridine dans 5 cm³ de dichlorométhane. On isole, selon l'exemple cité ci-dessus. On obtient 8 g d'une huile épaisse de couleur brun-jaunâtre. On traite la solution de celle-ci dans 50 cm³ de toluène par 5 cm³ d'oxychlorure de phosphore. On purifie le produit issu de la réaction par chromatographie sur une colonne de 2,8 cm de diamètre contenant 150 g de gel de silice (50 à 200 µ). On élue, en recueillant des fractions de 50 cm³, par 0,5 litre d'un mélange à parties égales (vol./vol.) d'oxyde de diisopropyle et d'hexane. On concentre à sec les fractions comprises entre 0,2 à 0,5 litre et on obtient 7 g d'une huile jaune claire. On dissout celle-ci dans 50 cm³ d'éthanol, ajoute une solution de 1,5 g de cyanure de potassium dans 10 cm³ d'eau et porte le mélange 6 heures au reflux. On chromatographie le produit de la réaction sur une colonne de 2,8 cm de diamètre contenant 120 g de gel de silice (50 à 200 µ). On élue, en recueillant des fractions de 100 cm³, d'abord avec 1,6 litre d'un mélange à parties égales de diisopropyle oxyde et d'hexane puis avec 0,5 litre d'oxyde de diéthyle. On concentre à sec les fractions provenant de ce dernier solvant et on obtient 4,3 g (55 %) de [(chloro-4 phényl)-4 (méthoxy-4 phényl)-5 oxazolyl-2]-7 heptanenitrile, sous la forme d'un solide fondant à 35°C.

### EXEMPLE 7

Selon l'exemple 5, on chauffe 4 g de [(chloro-4 phényl)-4 (méthoxy-4 phényl)-5 oxazolyl-2]-6 hexanenitrile, deux fois 2 g d'azoture de sodium et deux fois 1,7 g de chlorure d'ammonium dans 50 cm³ de diméthylformamide. On purifie le produit de la réaction par chromatographie sur une colonne de 2 cm de diamètre contenant 40 g de gel de silice (50 à 200 µ). On élue, en recueillant des fractions de 25 cm³, d'abord avec 0,2 litre d'oxyde de diisopropyle puis avec 0,3 litres d'acétate d'éthyle. On concentre à sec les éluats de l'ester acétique et cristallise le résidu solide dans 40 cm³ d'éthanol. On obtient 2 g (45 %) de {[(chloro-4 phényl)-4 (méthoxy-4 phényl)-5 oxazolyl-2]-5 pentyl}-5 tétrazole : cristaux blancs fondant à 160°C.

On peut préparer le [(chloro-4 phényl)-4 (méthoxy-4 phényl)-5 oxazolyl-2]-6 hexanenitrile de la manière suivante :
On chauffe 6 heures à 80°C, 8 g de (bromo-6 hexanamido)-2 (chloro-4 phényl)-2 (méthoxy-4 phényl)-1 éthanone et 5 cm³ d'oxychlorure de phosphore dans 100 cm³ de toluène. On concentre à sec sous pression réduite, ajoute 50 cm³ d'eau glacée et 150 cm³ d'oxyde de diéthyle. On lave l'extrait éthéré avec 100 cm³ de bicarbonate de sodium en solution aqueuse saturée. Après séchage sur sulfate de magnésium et concentration à sec sous vide, on obtient 7 g d'une huile jaune-clair. On dissout cette dernière dans 50 cm³ d'éthanol, ajoute 1,5 g de cyanure de potassium dans 5 cm³ d'eau. On chauffe 6 heures au reflux puis concentre à sec et ajoute 50 cm³ d'eau et 100 cm³ d'oxyde de diéthyle. On sèche sur sulfate de magnésium la phase organique et concentre à sec. On cristallise le résidu dans 30 cm³ d'hexane. On obtient 5 g de cristaux blancs fondant à 50°C.

On peut préparer la (bromo-6 hexanamido)-2 (chloro-4 phényl)-2 (méthoxy-4 phényl)-1 éthanone, selon l'exemple 6:
A partir de 6,2 g du chlorhydrate de l'amino-2 (chloro-4 phényl)-2 (méthoxy-4 phényl)-1 éthanone [préparé selon G. DREFAHL et coll., J. Prakt. Chem. 32, 307 (1966)] dans 50 cm³ de chlorure de méthylène contenant 4,5 g de chlorure de bromo-6 hexanoyle et 4 g de pyridine dans 5 cm³ de chlorure de méthylène, on obtient 8,5 g (93 %) de (bromo-6 hexanamido)-2 [chloro-4 phényl)-2 (méthoxy-4 phényl)-1 éthanone, cristaux blancs fondant à 96°C.

### EXEMPLE 8

Selon l'exemple 7, on chauffe 15 g de [(chloro-4 phényl)-5 (méthoxy-4 phényl)-4 oxazolyl-2]-6 hexanenitrile, deux fois 7,5 g d'azoture de sodium et 2 fois 6,2 g de chlorure d'ammonium dans 150 cm³ de diméthylformamide. On purifie le produit de la réaction par chromatographie sur une colonne de 2 cm de diamètre contenant 150 g de gel de silice. On élue, en recueillant des fractions de 50 cm³, par 0,8 litres d'acétate d'éthyle. On concentre à sec les fractions comprises entre 0,2 et 0,8 litre, cristallise dans 70 cm³ d'acétate d'isopropyle et obtient 4,5 g (27 %) de {[(chloro-4 phényl)-5 (méthoxy phényl)-4 oxazolyl-2]-6 hexyl}-5 tétrazole, cristaux lancs fondant à 122°C.

On peut préparer le [(chloro-4 phényl)-5 (méthoxy-4 phényl)-4 oxazolyl-2]-7 heptanenitrile, selon l'exemple 6 :
A partir de 35,5 g de chlorhydrate d'amino-2 (chloro-4 phényl)-1 (méthoxy-4 phényl)-2 éthanone dans 150 cm³ de dichlorométhane contenant 27,2 g de chlorure de bromo-7 heptanoyle, on ajoute 22,5 g de pyridine dans 30 cm³ de dichlorométhane. On purifie le produit de la réaction par chromatographie sur une colonne de 2,8 cm de diamère contenant 300 g de gel de silice. On élue en recueillant des fractions de 50 cm³, avec 1,2 litre de diisopropyle oxyde. On concentre à sec les fractions comprises entre 0,2 et 1,2 litre et on obtient 28,5 g d'une huile jaunâtre. On dissout celle-ci dans 115 cm³ de toluène et traite par 33 g d'oxychlorure de phosphore. On chromatographie le produit de la réaction sur une colonne de 2,8 cm de diamètre contenant 150 g de gel de silice. On élue, en recueillant des fractions de 50 cm³, avec 0,8 litres d'oxyde de diisopropyle. On concentre à sec sous pression réduite les fractions comprises entre 0,3 et 0,8 litre. On obtient 18 g d'une huile jaunâtre.

On ajoute à cette huile 100 cm³ d'éthanol et une solution de 8 g de cyanure de potassium dans 20 cm³ d'eau. On chauffe 22 heures le mélange à reflux. On chromatographie le produit de la réaction sur une colonne de 2 cm de diamètre contenant 150 g de gel de silice.

On élue, en recueillant des fractions de 50 cm³, avec 0,9 litre d'oxyde de diisopropyle. On concentre à sec sous pression réduite les fractions comprises entre 0,25 et 0,9 litre et on obtient 15 g d'une huile jaune clair, directement utilisée ci-dessus pour la synthèse du {[chloro-4 phényl)-5 (méthoxy-4 phényl)-4 oxazolyl-2]-6 hexyl}-5 tétrazole.

On peut préparer le chlorhydrate de l'amino-2 (chloro-4 phényl)-1 (méthoxy-4 phényl)-2 éthanone de la manière suivante :
On chauffe 7 heures à reflux, 45 g d'acétylamino-2 (chloro-4 phényl)-1 (méthoxy-4 phényl)-2 éthanone en solution dans 300 cm³ d'éthanol contenant 135 cm³ de l'acide chlorhydrique (d 1,18). Après filtration sur KIESELGUHR et concentration à sec sous vide, on ajoute 200 cm³ d'acétone au résidu solide, essore et lave avec le même solvant, 2 fois 75 cm³. On obtient 36 g (80 %) de hlorhyrate d'amino-2 (chloro-4 phényl)-1 (méthoxy-4 phényl)-2 éthanone, poudre blanche amorphe, fondant à 230°C.

On peut préparer l'acétylamino-2 (chloro-4 phényl)-1 (méthoxy-4 phényl)-2 éthanone, à partir de la (chloro-4 phényl)-1 (méthoxy-4 phényl)-2 éthanone (G.G. SMITH, F.D. BAGLEY et R. TAYLOR, J. Amer. Chem. Soc., 1961, 83 p. 3647), de la manière suivante : Selon le procédé de nitrosation d'une cétone par un nitrite d'alcoyle, en présence d'un alcoolate (C.F. KOELSCH et C.D. LE CLAIRE, J. Org. Chem., 6, 531 (1941), on ajoute à 20°C une solution de 40 g de (chloro-4 phényl)-1 (méthoxy-4 phényl)-2 éthanone dans 160 cm³ d'éthanol à une solution d'éthylate de sodium (préparée à partir de 7,8 g de sodium et 240 cm³ d'éthanol). Après agitation du mélange pendant 1 heure, on ajoute 38,8 g de nitrite de butyle, de manière à maintenir la réaction exothermique entre 30 et 40°C, puis on continue l'agitation 16 heures à la température ambiante. On verse le mélange dans 1,2 litre d'eau contenant 21 cm³ d'acide acétique. On extrait avec du chlorure de méthylène, 3 fois 150 cm³, lave les extraits organiques réunis avec 120 cm³ d'une solution saturée de bicarbonate de sodium puis à l'eau 2 fois 60 cm³, sèche sur sulfate de magnésium et évapore le solvant sous vide. On obtient 44 g d'un résidu pâteux, jaunâtre, directement utilisé dans la réaction suivante : Selon une méthode décrite pour transformer la benzile-mono-oxime en acétamidodésoxybenzoïne (H.O. HOUSE et W.F. BERKOWITZ, J. Org. Chem., 1963, 28, p. 307), on dissout le produit (44 g) de la réaction précédente dans 200 cm³ d'acide acétique contenant 216 g d'anhydride acétique. A la solution agitée, on ajoute en 2 heures 40 g de zinc en poudre fine (44 µ) de manière à maintenir la réaction exothermique entre 40 et 50°C. Après 4 heures d'agitation à 20°C, on filtre sur verre fritté. On concentre le filtrat à moitié, ajoute 400 cm³ d'éthanol et porte au reflux 1 heure. On concentre à sec sous pression réduite, dissout le résidu dans 800 cm³ de chlorure de méthylène et lave la solution organique avec une solution saturée de bicarbonate de sodium 3 fois 150 cm³, avec 100 cm³ d'eau, sèche sur sulfate de magnésium et concentre à sec sous pression réduite. On cristallise le résidu solide dans 290 cm³ d'éthanol. On obtient 46 g (94 %) d'acétylamino-2 (chloro-4 phényl)-1 (méthoxy-4 phényl)-2 éthanone, sous la forme de cristaux blancs fondant à 140°C.

### EXEMPLE 9:

Selon les conditions (temps et température) définies dans l'exemple 1, on traite 10 g de [bis(méthoxy-4 phényl)-4,5 oxazolyl-2]-7 méthyl-2 heptanenitrile-(R,S), dans 50 cm³ de diméthylformamide par 2 fois 4,8 g d'azoture de sodium et 2 fois 10,2 g de chlorhydrate de triéthylamine. Après traitement, on chromatographie le produit de la réaction sur une colonne de 2,8 cm de diamètre, contenant 90 g de gel de silice (50 à 200 µ). On élue par 1,8 litre d'oxyde de diisopropyle puis par 2,2 litres d'un mélange d'oxyde de diisopropyle-acétate d'éthyle (30-70 en volume), en recueillant des fractions de 200 cm³. On concentre à sec les fractions du dernier mélange éluant sous pression réduite (0,2 kPa). On dissout le résidu obtenu (8,4 g) dans 178 cm³ de soude 0,1 N, concentre à sec sous pression réduite (0,2 kPa). Le produit obtenu est mis en suspension dans 50 cm³ d'oxyde de diisopropyle sous agitation, essoré, lavé par 2 fois 20 cm³ d'oxyde de diisopropyle, et séché. On obtient 8,1 g (69 %) de sel de sodium de {[bis(méthoxy-4 phényl)-4,5 oxazolyl-2]-7 heptyl-2}-5 1H-tétrazole-(R,S), sous la forme de cristaux blancs fondant à 104°C.

Le [bis(méthoxy-4 phényl)-4,5 oxazolyl-2]-7 méthyl-2 heptanenitrile (R,S) peut être préparé comme suit:
A une solution de 13,8 g de bis(méthoxy-4 phényl)-1,2 (cyano-7 octanamido)-2 éthanone (R,S) dans 65 cm³ de toluène, on ajoute, sous agitation 15 g d'oxychlorure de phosphore et on chauffe le mélange 4 heures à 80°C. On verse la solution sur 50 g de glace, décante la phase toluènique, extrait la phase aqueuse par 2 fois 50 cm³ d'acétate d'éthyle. Les extraits organiques réunis sont lavés à l'eau (3 fois 20 cm³) et par 30 cm³ d'une solution aqueuse saturée en chlorure de sodium. Après séchage sur sulfate de sodium, le solvant est évaporé sous pression réduite. Le résidu est chromatographié sur une colonne de 2,8 cm de diamètre, contenant 100 g de gel de silice (50 à 200 ). On élue, en recueillant des fractions de 50 cm³, par 1,8 litre d'oxyde de diisopropyle. On concentre sous pression réduite (0,2 kPa) les fractions comprises entre 0,3 litre et 1,8 litre. On obtient 12,4 g (93 %) de [bis(méthoxy-4 phényl)-4,5 oxazolyl-2]-7 méthyl-2 heptanenitrile (R,S) sous la forme d'une huile jaunâtre directement utilisée dans la réaction ultérieure.

Spectre de RMN du proton (300 MHz, CDCl₃ δ en ppm, J en Hz) :
1,3 (d, J=6,5, 3 H, -CH₃ )
1,35 à 1,75 (mt, 6H, =CH₂ centraux)
1,87 (mt, 2H, =CH₂ en β de l'oxazole)
2,6 (mt, 1H, -CH<)
2,83 (t, J=7, 2H, =CH₂ en α de l'oxazole)
3,84 (s, 6H, -OCH₃)
6,91 (d, J=8,5, 4H, aromatiques en ortho des -OCH₃)
7,51 et 7,56 (2d, J=8,5, 4H, aromatiques )
La bis(méthoxy-4 phényl)-1,2 (cyano-7 octanamido)-2 éthanone (R,S) peut être préparée comme suit:
On ajoute 14,4 g de sel de dicyclohexylamine de l'acide cyano-7 octanoïque à un mélange de 45 cm³ d'une solution molaire de sulfate acide de sodium et 100 cm³ d'oxyde de diéthyle. On agite 15 minutes à 20°C puis on sépare la phase organique. On sèche celle-ci sur sulfate de magnésium, filtre, et évapore le solvant sous pression réduite. Le résidu, additionné de 24,6 g de chlorure de thionyle est chauffé 3 heures à 50°C. Le mélange est évaporé sous pression réduite. L'huile résiduelle est dissoute dans 10 cm³ de dichlorométhane, la solution obtenue est ajoutée à une suspension agitée et refroidie à 5°C de 12,1 g de chlorhydrate d'amino-2 bis(méthoxy-4 phényl)-1,2 éthanone dans 50 cm³ de dichlorométhane contenant 7,8 g de pyridine. On agite le mélange 20 heures à la température ambiante puis on ajoute 40 cm³ d'eau, décante la phase organique, lave celle-ci avec 20 cm³ d'acide chlorhydrique normal et à l'eau (3 fois 20 cm³). On sèche sur sulfate de magnésium, filtre et évapore le solvant sous pression réduite. Le résidu est chromatographié sur une colonne de 2,8 cm de diamètre, contenant 100 g de gel de silice (50 à 200 µ) . On élue, en recueillant des fractions de 200 cm³, par 4 litres d'un mélange d'oxyde de diisopropyle-acétate d'éthyle (80-20 en volume). On concentre sous pression réduite (0,2 kPa) les fractions comprises entre 0,6 litre et 4 litres. On obtient 13,9 g (83 %) de bis(méthoxy-4 phényl)-1,2 (cyano-7 octanamido)-2 éthanone (R,S), sous la forme d'une huile jaunâtre directement utilisée dans la réaction ultérieure.

Spectre de RMN du proton (200 MHz, CDCl₃, δ en ppm, J en Hz):
1,28 (d, J=7, 3 H, -CH₃ )
1,25 à 1,75 (mt, 8H, =CH₂)
2,24 (t, J=7,5, 2H, =NCO-CH₂-)
2,55 (mt, 1H, -CH<)
3,74 et 3,81 (2s, 3H chacun, -OCH₃)
6,46 (d, J=7, 1H, -CO-CH-N-CO-)
6,82 et 6,87 (2d, J=9, 4H, aromatiques en ortho des -OCH₃)
6,99 (d large, J=7, -CONH-)
7,3 (d, J=9, 2H, aromatiques )
7,95(d, J=9, 2H, aromatiques en ortho du C=O)
Le sel de dicyclohexylamine de l'acide cyano-7 octanoïque peut être préparé comme suit:
On dissout 100 g de bromo-6 hexanoate d'éthyle, 253,4 g de cyanacétate d'éthyle dans 900 cm³ de diméthylformamide et on ajoute au mélange 61,9 g de carbonate de potassium et 16,5 g d'iodure de tétrabutylammonium. On agite la suspension à 60°C pendant 5 heures. Après refroidissement, on ajoute 2,7 litres d'eau, extrait avec de l'oxyde de diéthyle (3 fois 200 cm³). On lave les extraits éthérés réunis avec 3 fois 100 cm³ d'eau, puis par 100 cm³ d'une solution aqueuse saturée en chlorure de sodium. Après séchage sur sulfate de magnésium, le solvant est évaporé sous pression réduite. Le résidu de l'évaporation est distillé sous pression réduite (0,01 kPa) à 144°C. On obtient 95,1 g (83 %) d'une huile incolore. On dissout celle-ci dans 740 cm³ de diméthylformamide et on ajoute au mélange 51,1 g de carbonate de potassium, 13,7g d'iodure de tétrabutylammonium et 105 g d'iodure de méthyl. On agite la suspension à 20°C pendant 18 heures, ajoute 2,2 litres d'eau et extrait avec de l'oxyde de diéthyle (3 fois 200 cm³). On lave les extraits éthérés réunis avec 6 fois 100 cm³ d'eau, puis par 100 cm³ d'une solution aqueuse saturée en chlorure de sodium. Après séchage sur sulfate de magnésium, le solvant est évaporé à sec. Le résidu de l'évaporation est distillé sous pression réduite (0,01 kPa) à 130°C. On obtient 93,3 g (93 %) d'une huile incolore. On dissout celle-ci dans 430 cm³ de d'éthanol et on ajoute une solution de 56,5 g de potasse à 86% dans 40 cm³ d'eau. On agite le mélange 48 heures à 20°C, acidifie celui-ci par 74 cm³ d'acide chlorhydrique 12 N et concentre à sec sous pression réduite. On ajoute au résidu 40 cm³ d'eau, extrait avec de l'oxyde de diéthyle (3 fois 100 cm³), lave les extraits éthérés réunis avec 3 fois 30 cm³ d'eau, sèche sur sulfate de magnésium, filtre et concentre à sec. Le résidu de l'évaporation est chauffé à 160°C pendant 3 heures, jusqu'à cessation du dégagement de gaz carbonique puis on distille le produit sous pression réduite (0,01 kPa) entre 205 et 210°C. On obtient 46,8 g d'acide qui est mis en solution dans 250 cm³ d'oxyde de diéthyle avec 37,6 g de dicyclohexylamine. On laisse sous agitation 1 heure et on essore le précipité. Le filtrat est additionné de nouveau par 50 g de dicyclohexylamine, laissé une nuit au repos. Le sel de dicyclohexylamine qui précipite est essoré, lavé par 2 fois 50 cm³ d'oxyde de diéthyle et séché. On obtient 51,4 g (42 %) de sel de dicyclohexylamine de l'acide cyano-7 octanoïque sous la forme d'une poudre blanche fondant à 85°C.

### EXEMPLE 10:

Selon les conditions (temps et température) définies dans l'exemple 1, on traite 4,45 g de [bis(chloro-4 phényl)-4,5 oxazolyl-2]-7 méthyl-2 heptanenitrile-(R,S), dans 40 cm³ de diméthylformamide par 2 fois 2,1 g d'azoture de sodium et 2 fois 4,4 g de chlorhydrate de triéthylamine. Après traitement, on chromatographie le produit de la réaction sur une colonne de 2,8 cm de diamètre, contenant 115 g de gel de silice (50 à 200 µ). On élue par 1,2 litre d'oxyde de diisopropyle puis par 2,8 litres d'un mélange d'oxyde de diisopropyle-acétate d'éthyle (80-20 en volume), en recueillant des fractions de 200 cm³. On concentre à sec les fractions du dernier mélange éluant sous pression réduite (0,2 kPa). On obtient 2,7 g (55 %) de {[bis(chloro-4 phényl)-4,5 oxazolyl-2]-7 heptyl-2}-5 1 H-tétrazole-(R,S), sous la forme de cristaux blancs fondant à 144°C.

On peut préparer le [bis(chloro-4 phényl)-4,5 oxazolyl-2]-7 méthyl-2 heptanenitrile (R,S) selon les conditions définies dans l'exemple 9:
A une solution de 7,5 g de bis(chloro-4 phényl)-1,2 (cyano-7 octanamido)-2 éthanone (R,S) dans 35 cm³ de toluène, on ajoute, sous agitation 8 g d'oxychlorure de phosphore et on chauffe le mélange 6 heures à 90°C. Après traitement, on chromatographie le produit obtenu sur une colonne de 2,8 cm de diamétre, contenant 110 g de gel de silice (50 à 200 µ). On élue par 0,6 litre d'oxyde de diisopropyle, en recueillant des fractions de 50 cm³. On concentre sous pression réduite (0,2 kPa) les fractions entre 0,15 litre et 0,6 litre. On obtient 6,6 g (91 %) de [bis(chloro-4 phényl)-4,5 oxazolyl-2]-7 méthyl-2 heptanenitrile (R,S) sous la forme d'une huile jaunâtre directement utilisée dans la réaction ultérieure.

Spectre de RMN du proton (300 MHz, CDCl₃, δ en ppm, J en Hz) :
1,32 (d, J=7, 3 H, -CH₃ )
1,4 à 1,8 (mt, 6H, =CH₂ centraux)
1,87 (mt, 2H, =CH₂ en β de l'oxazole)
2,61 (mt, 1H, -CH<)
2,85 (t, J=7, 2H, =CH₂ en α de l'oxazole)
7,34 (d, J=8,5, 4H, aromatiques en ortho des Cl)
7,49 et 7,54 (2d, J=8,5, 4H, aromatiques )
On peut préparer la bis(chloro-4 phényl)-1,2 (cyano-7 octanamido)-2 éthanone (R,S) selon les conditions définies dans l'exemple 9:
On ajoute 8,2 g de sel de dicyclohexylamine de l'acide cyano-7 octanoïque à un mélange de 26 cm³ d'une solution molaire de sulfate acide de sodium et 60 cm³ d'oxyde de diéthyle. On agite 15 minutes à 20°C puis on sépare la phase organique. On sèche celle-ci sur sulfate de magnésium, filtre et évapore le solvant sous pression réduite. Le résidu, additionné de 13,2 g de chlorure de thionyle est chauffé 3 heures à 50°C. Le mélange est concentré sous pression réduite. L'huile résiduelle est dissoute dans 5 cm³ de dichlorométhane, la solution obtenue est ajoutée à une suspension agitée et refroidie à 5°C de 7 g de chlorhydrate d'amino-2 bis(chloro-4 phényl)-1,2 éthanone dans 25 cm³ de dichlorométhane contenant 4,4 g de pyridine. Après traitement, on chromatographie le produit obtenu sur une colonne de 2,8 cm de diamètre, contenant 140 g de gel de silice (50 à 200 µ). On élue par 1 litre d'oxyde de diisopropyle puis par 2,5 litres d'un mélange d'oxyde de diisopropyle-acétate d'éthyle (90-10 en volume), en recueillant des fractions de 250 cm³. On concentre à sec sous pression réduite (0,2 kPa), les fractions du dernier mélange éluant. On obtient 7,6 g (80 %) de bis(chloro-4 phényl)-1,2 (cyano-7 octanamido)-2 éthanone (R,S) sous la forme d'une huile jaunâtre directement utilisée dans la réaction ultérieure.

Spectre de RMN du proton (300 MHz, CDCl₃, δ en ppm et J en Hz) :
1,26 (d, J=7, 3 H, -CH₃ )
1,2 à 1,7 (mt, 8H, =CH₂ centraux)
2,22 (t, J=7,5, 2H, =NCO-CH₂-)
2,51 (mt, 1H, -CH<)
6,44 (d, J=7, 1H, -CO-CH-N-CO-)
6,90 (d large, J=7, 1H, -CONH-)
7,25 (d, J=8,5, 4H, aromatiques en ortho des Cl)
7,35 (d, J=8,5,2H, aromatiques )
7,85 (d, J=8,5, 2H, aromatiques en ortho du C=O)

### EXEMPLE 11:

Selon les conditions (temps et température) définies dans l'exemple 1, on traite 3 g de [diphényl-4,5 oxazolyl-2]-7 méthyl-2 heptanenitrile-(R,S), dans 30 cm³ de diméthylformamide par 2 fois 1,7 g d'azoture de sodium et 2 fois 3,6 g de chlorhydrate de triéthylamine. Après traitement, on chromatographie le produit de la réaction sur une colonne de 2 cm de diamètre, contenant 78 g de gel de silice (50 à 200 µ). On élue, en recueillant des fractions de 200 cm³, par 1 litre d'oxyde de diisopropyle puis par 1,6 litre d'un mélange d'oxyde de diisopropyle-acétate d'éthyle (95-05 en volume). On concentre à sec les fractions du dernier mélange éluant, sous pression réduite (0,2 kPa). On obtient 2,3 g (68 %) de {[diphényl-4,5 oxazolyl-2]-7 heptyl-2}-5 1H-tétrazole-(R,S), sous la forme d'une huile jaunâtre.

Spectre de RMN du proton (200 MHz, CDCl₃, δ en ppm et J en Hz):
1,37 (d, J=7, 3 H, -CH₃ )
1,1 à 1,95 (mt, 8H, =CH₂ centraux)
2,85 (t, J=7,5, 2H, =CH₂ en α de l'oxazole)
3,17 (mt, 1H, -CH<)
7,35 (mt, 6H, aromatiques )
7,57 (mt, 4H, aromatiques en ortho des substitutions)
On peut préparer le [diphényl-4,5 oxazolyl-2]-7 méthyl-2 heptanenitrile (R,S) selon les conditions définies dans l'exemple 9:
A une solution de 5,75 g de diphényl-1,2 (cyano-7 octanamido)-2 éthanone (R,S) dans 30 cm³ de toluène, on ajoute, sous agitation 7,3 g d'oxychlorure de phosphore et on chauffe le mélange 6 heures à 80°C. Après traitement, on chromatographie le produit obtenu sur une colonne de 2 cm de diamètre, contenant 60 g de gel de silice (50 à 200 µ). On élue par 0,6 litre d'oxyde de diisopropyle, en recueillant des fractions de 50 cm³. On concentre sous pression réduite (0,2 kPa) les fractions comprises entre 0,1 litre et 0,6 litre. On obtient 4,2 g (76 %) de [diphényl-4,5 oxazolyl-2]-7 méthyl-2 heptanenitrile (R,S) sous la forme d'une huile directement utilisée dans la réaction ultérieure.

Spectre de RMN du proton (200 MHz, CDCl₃, δ en ppm et J en Hz):
1,34 (d, J=6,5, 3 H, -CH₃ )
1,4 à 2 (mt, 8H, =CH₂ centraux)
2,64 (mt, 1H, -CH<)
2,9 (t, J=7,5, 2H, =CH₂ en α de l'oxazole)
7,4 (mt, 6H, aromatiques )
7,6 et 7,65 (2d, J=8,5, 4H, aromatiques )
On peut préparer la diphényl-1,2 (cyano-7 octanamido)-2 éthanone (R,S) selon les conditions définies dans l'exemple 9:
On ajoute 9 g de sel de dicyclohexylamine de l'acide cyano-7 octanoïque à un mélange de 28 cm³ d'une solution molaire de sulfate acide de sodium et 60 cm³ d'oxyde de diéthyle. On agite 15 minutes à 20°C puis on sépare la phase organique. On sèche celle-ci sur sulfate de magnésium, filtre et évapore le solvant sous pression réduite. Le résidu, additionné de 14,8 g de chlorure de thionyle est chauffé 3 heures à 50°C. Le mélange est concentré sous pression réduite. L'huile résiduelle est dissoute dans 6 cm³ de dichlorométhane, la solution obtenue est ajoutée à une suspension agitée et refroidie à 5°C de 6 g de chlorhydrate d'amino-2 diphényl-1,2 éthanone dans 30 cm³ de dichlorométhane contenant 4,8 g de pyridine. Après traitement, on chromatographie le produit obtenu sur une colonne de 2 cm de diamètre, contenant 48 g de gel de silice (50 à 200 µ). On élue, en recueillant des fractions de 100 cm³, par 1,2 litre d'un mélange d'oxyde de diisopropyle-acétate d'éthyle (80-20 en volume). On concentre sous pression réduite (0,2 kPa) les fractions comprises entre 0,3 et 1,2 litre. On obtient 5,9 g (67 %) de diphényl-1,2 (cyano-7 octanamido)-2 éthanone (R,S) sous la forme d'une huile directement utilisée dans la réaction ultérieure.

Spectre de RMN du proton (200 MHz, CDCl₃, δ en ppm et J en Hz):
1,2 (d, J=7, 3 H, -CH₃ )
1,1 à 1,7 (mt, 8H, =CH₂ centraux)
2,18 (t, J=7,5, 2H, =NCO-CH₂-)
2,48 (mt, 1H, -CH<)
6,5 (d, J=7, 1H, -CO-CH-N-CO-)
6,95 (d large, J=7, 1H, -CONH-)
7,05 à 7,55 (mt, 8H, aromatiques )
7,9 (d, J=8,5, aromatiques en ortho du C=O)

### EXEMPLE 12:

Selon les conditions (temps et température) définies dans l'exemple 1, on traite 5 g de [bis(méthoxy-4 phényl)-4,5 oxazolyl-2]-7 diméthyl-2,2 heptanenitrile, dans 12 cm³ de diméthylformamide par 2 fois 2,27 g d'azoture de sodium et 2 fois 4,95 g de chlorhydrate de triéthylamine. Après traitement, on chromatographie le produit de la réaction sur une colonne de 3,5 cm de diamètre, contenant 120 g de gel de silice (50 à 200 µ). On élue, en recueillant des fractions de 50 cm³, par 0,5 litre d'oxyde de diisopropyle puis par 1,3 litre d'un mélange d'oxyde de diisopropyle-acétate d'éthyle (70-30 en volume). On concentre sous pression réduite (0,2 kPa) les fractions comprises entre 0,85 litre et 1,3 litre. Le solide repris par 30 cm³ d'oxyde de diéthyle est essoré, lavé avec 10 cm³ d'oxyde de diéthyle, et séché. On obtient 2,8 g (50 %) de {[bis(méthoxy-4 phényl)-4,5 oxazolyl-2]-7 méthyl-2 heptyl-2}-5 1H-tétrazole, sous la forme de cristaux blancs fondant à 82°C.

Le [bis(méthoxy-4 phényl)-4,5 oxazolyl-2]-7 diméthyl-2,2 heptanenitrile peut être préparé comme suit:
On maintient 1 heure à -40°C le mélange de 2,9 g d'isobutyronitrile dans 35 cm³ de tétrahydrofuranne et de 21 cm³ d'une solution 2 fois molaire de diisopropylamidure de lithium dans l'hexane. La solution est refroidie à -70°C puis additionnée en 20 minutes d'une solution de 15 g de bis(méthoxy-4 phényl)-4,5 (bromo-5 pentyl)-2 oxazole (préparé comme à l'exemple 1) dans 30 cm³ de tétrahydrofuranne. La solution, ramenée en 2 heures à température ambiante, est reprise par 150 cm³ d'eau puis par 200 cm³ d'oxyde de diéthyle. La phase organique est décantée, séchée sur sulfate de magnésium, filtrée et concentrée à sec sous pression réduite. Le résidu est chromatographié sur une colonne de 2,8 cm de diamètre, contenant 100 g de gel de silice (50 à 200 µ). On élue, en recueillant des fractions de 50 cm³, par 0,7 litre d'oxyde de diisopropyle. On concentre sous pression réduite (0,2 kPa) les fractions comprises entre 0,35 litre et 0,7 litre. On obtient 6 g (42 %) de [bis (méthoxy-4 phényl)-4,5 oxazolyl-2]-7 diméthyl-2,2 hepta-nenitrile, sous la forme d'une huile jaunâtre direc-tement utilisée dans la réaction ultérieure.

Spectre de RMN du proton (300 MHz, CDCl₃, δ en ppm et J en Hz):
1,35 (s, 6H, =C(CH₃)₂ )
1,4 à 1,65 (mt, 6H, =CH₂ centraux)
1,87 (mt, 2H, =CH₂ en β de l'oxazole)
2,85 (t, J=7,5, 2H, =CH₂ en α de l'oxazole)
3,85 (s, 6H, -OCH₃)
6,90 (d, J=9, 4H, aromatiques en ortho des -OCH₃)
7,5 et 7,55 (2d, J=9, 4H, aromatiques)
La présente invention concerne également les compositions pharmaceutiques constituées par un produit de formule générale (I), ou un sel, éventuellement en association avec tout autre produit compatible, pouvant être inerte ou physiologiquement actif. Les compositions selon l'invention peuvent être utilisées par voie parentérale, orale, rectale ou topique.

Comme compositions solides pour administration orale, peuvent être utilisés des comprimés, des pilules, des poudres ou des granulés. Dans ces compositions, le produit actif selon l'invention est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration parentérale, peuvent être des solutions stériles aqueuses ou non aqueuses, des suspensions ou des émulsions. Comme solvant ou véhicule, on peut employer le propylèneglycol, un polyéthylèneglycol, des huiles végétales, en particulier l'huile d'olive, et des esters organiques injectables, par exemple l'oléate d'éthyle. Ces compositions peuvent également contenir des adjuvants, en particulier des agents mouillants, émulsifiants ou dispersants. La stérilisation peut se faire de plusieurs façons, par exemple à l'aide d'un filtre bactériologique, en incorporant à la composition des agents stérilisants, par irradiation ou par chauffage. Elles peuvent également être préparées sous forme de compositions solides stériles qui seront dissoutes au moment de l'emploi dans de l'eau stérile ou tout autre milieu stérile injectable.

Les compositions pour àdministration rectale sont les suppositoires ou les capsules rectales qui peuvent contenir, outre le produit actif, des excipients tels que le beurre de cacao ou la suppo-cire.

Les compositions pour administration topique peuvent être par exemple des crèmes, des pommades, ou des lotions.

En thérapeutique humaine, les produits selon l'invention peuvent être particulièrement utiles dans le traitement des maladies d'origine inflammatoire. Ils peuvent donc s'avérer très utiles en pathologie ostéoarticulaire dans le traitement de l'arthrite, de la polyarthrite rhumatoïde, de la spondylarthrite , de la goutte, de l'arthrose, de la chondrocalcinose, ainsi que dans d'autres pathologies inflammatoires touchant les poumons, les voies digestives (colite ulcéreuse, inflammation hépatique, cirrhose, maladies du colon, maladie de Crohn), la peau (psoriasis, herpès, acné, érythème, eczéma, dermites), les yeux, les voies nasales, la cavité buccale et les dents. Ils peuvent également être utilisés dans les traitements des allergies nasales et bronchiques (asthme). Les produits selon l'invention peuvent aussi être utiles dans les traitements des inflammations liées à la mise en place d'implants, en améliorant leur compatibilité avec le tissu environnant. Ils peuvent également jouer un rôle dans l'immunorégulation (maladies auto-immunes), l'ischémie et la reperfusion (cardiaque notamment).

Ces produits exercent aussi un effet bénéfique dans le traitement de l'hyperthermie et de la douleur.

Les doses dépendent de l'effet recherché et de la durée du traitement. Pour un adulte elles sont généralement comprises entre 500 mg et 1 g par jour par voie orale. D'une manière générale, le médecin déterminera la dose qu'il estime la plus appropriée en fonction de l'âge, du poids et de tous les autres facteurs propres au sujet à traiter.

L'exemple suivant illustre une composition selon l'invention.

### EXEMPLE

On prépare selon la technique habituelle, des comprimés de produit actif ayant la composition suivante :

| | |
|---|---|
| - {[Bis(méthoxy-4 phényl)-4,5 oxazolyl-2]-6 hexyl}-5 tétrazole | 100 mg |
| - amidon | 332 mg |
| - silice | 120 mg |
| - stéarate de magnésium | 12 mg |

## Revendications (Revendications pour l'(les) Etat(s) contractant(s) suivant(s): AT, BE, CH, DE, DK, FR, GB, IT, LI, LU, NL, SE)

1. Un dérivé de l'oxazole de formule générale : 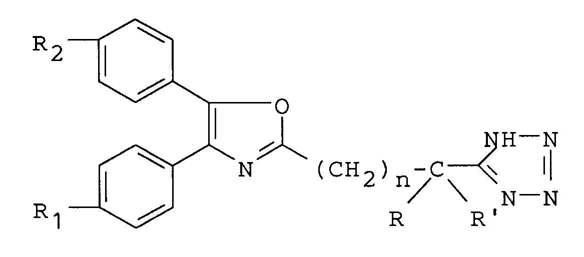 dans laquelle :
R et R' sont identiques ou différents et représentent un atome d'hydrogène ou un radical alcoyle contenant 1 ou 2 atomes de carbone, R₁ et R₂ sont identiques ou différents et représentent des atomes d'hydrogène ou d'halogène, ou des radicaux alcoyloxy dont la partie alcoyle contient 1 à 4 atomes de carbone en chaîne droite ou ramifiée, et
n égale 3 à 6,
ainsi que ses sels et, lorsqu'ils existent, ses isomères et leurs mélanges.

2. Un dérivé de l'oxazole selon la revendication 1, caractérisé en ce que
R et R' sont identiques ou differents et représentent un atome d'hydrogène ou un radical méthyle,
R₁ et R₂ sont identiques ou différents et représentent des atomes d'hydrogène ou d'halogène ou des radicaux méthoxy et
n égale 4 ou 5
ainsi que ses sels, ses isomères lorsqu'ils existent et leurs mélanges.

3. Un dérivé de l'oxazole selon l'une des revendications 1 ou 2, caractérisé en ce qu'il s'agit du {[bis (méthoxy-4 phényl)-4,5 oxazolyl-2]-6 hexyl-5 tétrazole ainsi que ses sels.

4. Un dérivé de l'oxazole selon l'une des revendications 1 ou 2, caractérisé en ce qu'il s'agit du {[bis(méthoxy-4 phényl)-4,5 oxazolyl-2]-7 heptyl-2}-5 1H-tétrazole ainsi que ses sels, ses isomères et leurs mélanges.

5. Un dérivé de l'oxazole selon l'une des revendications 1 ou 2, caractérisé en ce qu'il s'agit du {[bis(méthoxy-4 phényl)-4,5 oxazolyl-2]-7 méthyl-2 heptyl-2}-5 1H-tétrazole ainsi que ses sels.

6. Un dérivé de l'oxazole selon l'une des revendications 1 ou 2, caractérisé en ce qu'il s'agit du {[bis(méthoxy-4 phényl)-4,5 oxazolyl-2]-5 pentyl}-5 tétrazole ainsi que ses sels.

7. Un dérivé de l'oxazole selon l'une des revendications 1 ou 2, caractérisé en ce qu'il s'agit du {[(chloro-4 phényl)-4 (méthoxy-4 phényl)-5 oxazolyl-2]-6 hexyl}-5 tétrazole ainsi que ses sels.

8. Procédé de préparation d'un dérivé de l'oxazole selon la revendication 1, caractérisé en ce que l'on fait agir un azoture alcalin ou l'azoture de tri-n.butyl étain sur un nitrile de formule générale : 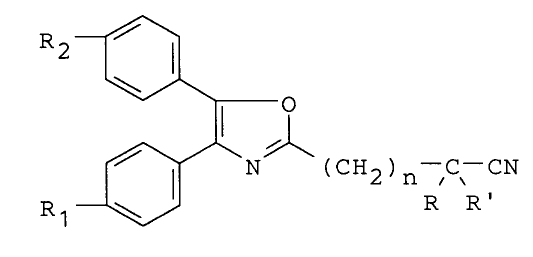 dans laquelle R, R' R₁, R₂ et n sont définis comme dans la revendication 1 puis transforme éventuellementle produit obtenu en son sel.

9. Procédé selon la revendication 8, caractérisé en ce que l'on opère en présence de chlorure d'ammonium ou du chlorhydrate d'une base organique azotée, lorsque l'on met en oeuvre un azoture alcalin.

10. Procédé selon la revendication 8, caractérisé en ce que l'azoture alcalin est l'azoture de sodium.

11. Composition pharmaceutique caractérisée en ce qu'elle contient au moins un produit selon la revendication 1, à l'état pur ou sous forme d'association avec un ou plusieurs diluants ou adjuvants compatibles et pharmaceutiquement acceptables.

## Revendications (Revendications pour l'(les) Etat(s) contractant(s) suivant(s): ES, GR)

1. Procédé de préparation d'un nouveau dérivé de l'oxazole de formule générale : 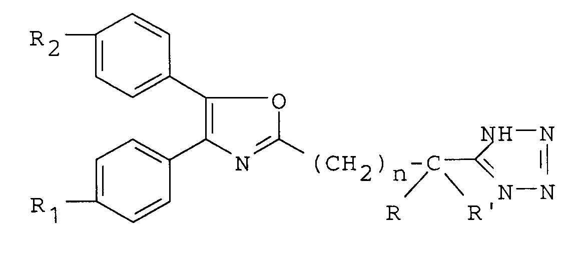 dans laquelle :
R et R' sont identiques ou différents et rprésentent un atome d'hydrogène ou un radical alcoyle contenant 1 ou 2 atomes de carbone, R₁ et R₂ sont identiques ou différents et représentent des atomes d'hydrogène ou d'halogène, ou des radicaux alcoyloxy dont la partie alcoyle contient 1 à 4 atomes de carbone en chaîne droite ou ramifiée, et
n égale 3 à 6,
ainsi que ses sels et, lorsqu'ils existent, ses isomères et leurs mélanges,
caractérisé en ce que l'on fait agir un azoture alcalin ou l'azoture de tri-n.butyl étain sur un nitrile de formule générale : 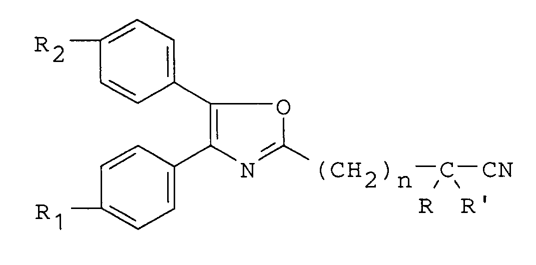 dans laquelle R, R' R₁, R₂ et n sont définis comme ci-dessus, puis transforme éventuellement le produit obtenu en son sel.

2. Procédé selon la revendication 1, caractérisé en ce que l'on opère en présence de chlorure d'ammonium ou du chlorhydrate d'une base organique azotée, lorsque l'on met en oeuvre un azoture alcalin.

3. Procédé selon la revendication 1, caractérisé en ce que l'azoture alcalin est l'azoture de sodium.

## Claims (Claims for the following Contracting State(s): AT, BE, CH, DE, DK, FR, GB, IT, LI, LU, NL, SE)

1. An oxazole derivative of general formula: 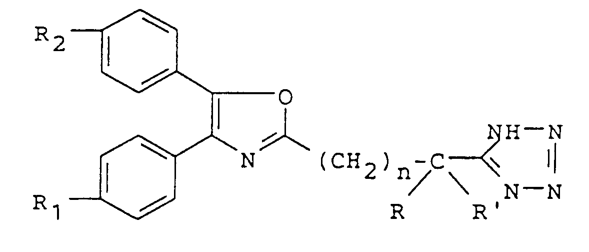 in which:
R and R' are identical or different and represent a hydrogen atom or an alkyl radical containing 1 or 2 carbon atoms, R₁ and R₂ are identical or different and represent hydrogen or halogen atoms or alkyloxy radicals in which the alkyl portion contains 1 to 4 carbon atoms in a straight or branched chain, and
n equals 3 to 6,
as well as its salts and, where they exist, its isomers and mixtures thereof.

2. An oxazole derivative according to Claim 1, characterized in that
R and R' are identical or different and represent a hydrogen atom or a methyl radical,
R₁ and R₂ are identical or different and represent hydrogen or halogen atoms or methoxy radicals, and n equals 4 or 5,
as well as its salts, its isomers where they exist and mixtures thereof.

3. An oxazole derivative according to one of claims 1 and 2, characterized in that it is 5-{6-[4,5-bis(4-methoxyphenyl)-2-oxazolyl]hexyl}tetrazole, as well as its salts.

4. An oxazole derivative according to one of Claims 1 and 2, characterized in that it is 5-{7-[4,5-bis(4-methoxyphenyl)-2-oxazolyl]-2-heptyl}-1H-tetrazole, as well as its salts, its isomers and mixtures thereof.

5. An oxazole derivative according to one of Claims 1 and 2, characterized in that it is 5-{7-[4,5-bis-(4-methoxyphenyl)-2-oxazolyl]-2-methyl-2-heptyl}-1H-tetrazole as well as its salts.

6. An oxazole derivative according to one of Claims 1 and 2, characterized in that it is 5-{5-[4,5-bis-(4-methoxyphenyl)-2-oxazolyl]pentyl}tetrazole, as well as its salts.

7. An oxazole derivative according to one of Claims 1 and 2, characterized in that it is 5-{6-[4-(4-chlorophenyl)-5-(4-methoxyphenyl)2-oxazolyl]hexyl}-tetrazole, as well as its salts.

8. Process for preparing an oxazole derivative according to Claim 1, characterized in that an alkali metal azide or tri-n-butyltin azide is reacted with a nitrile of general formula: 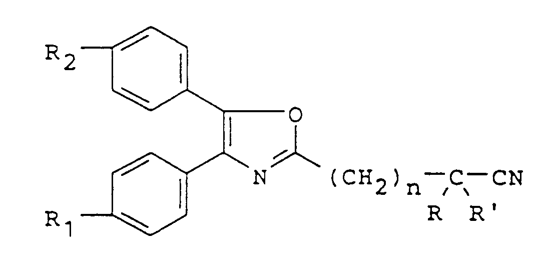 in which R, R', R₁, R₂ and n are defined as in Claim 1, and the product obtained is then optionally converted to its salt.

9. Process according to Claim 8, characterized in that the reaction is performed in the presence of ammonium chloride or of the hydrochloride of a nitrogenous organic base when an alkali metal azide is employed.

10. Process according to Claim 8, characterized in that the alkali metal azide is sodium azide.

11. Pharmaceutical composition, characterized in that it contains at least one product according to Claim 1, in the pure state or in the form of a combination with one or more compatible and pharmaceutically acceptable diluents or adjuvants.

## Claims (Claims for the following Contracting State(s): ES, GR)

1. Process for preparing a new oxazole derivative of general formula: 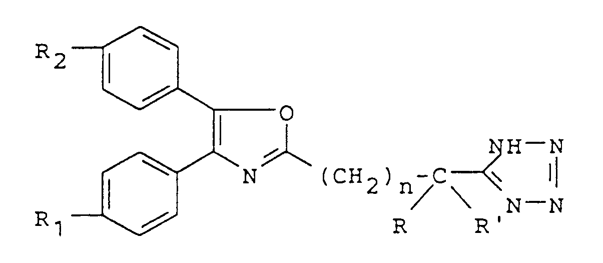 in which:
R and R' are identical or different and represent a hydrogen atom or an alkyl radical containing 1 or 2 carbon atoms, R₁ and R₂ are identical or different and represent hydrogen or halogen atoms or alkyloxy radicals in which the alkyl portion contains 1 to 4 carbon atoms in a straight or branched chain, and
n equals 3 to 6,
as well as its salts and, where they exist, its isomers and mixtures thereof,
characterized in that an alkali metal azide or tri-n-butyltin azide is reacted with a nitrile of general formula: 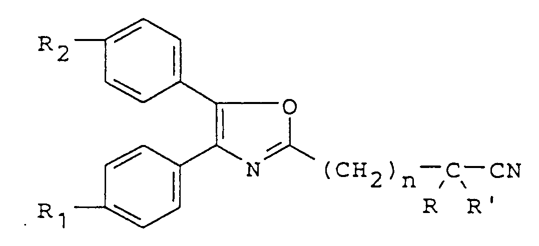 in which R, R', R₁, R₂ and n are defined as above, and the product obtained is then optionally converted to its salt.

2. Process according to Claim 1, characterized in that the reaction is performed in the presence of ammonium chloride or of the hydrochloride of a nitrogenous organic base when an alkali metal azide is employed.

3. Process according to Claim 1, characterized in that the alkali metal azide is sodium azide.

## Patentansprüche (Patentansprüche für folgende(n) Vertragsstaat(en): AT, BE, CH, DE, DK, FR, GB, IT, LI, LU, NL, SE)

1. Ein Oxazolderivat der allgemeinen Formel 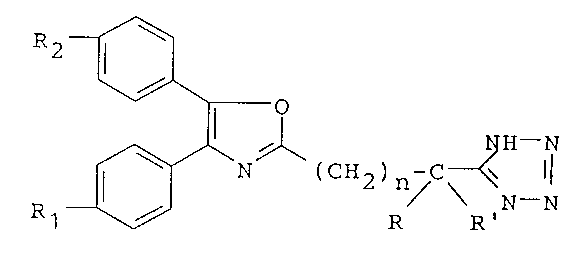 in der
R und R' gleich oder verschieden sind und ein Wasserstoffatom oder einen Alkylrest mit 1 oder 2 Kohlenstoffatomen darstellen,
R₁ und R₂ gleich oder verschieden sind und Wasserstoffatome oder Halogenatome oder Alkyloxyreste darstellen, deren Alkylteil 1 bis 4 Kohlenstoffatome in gerader oder verzweigter Kette enthält, und
n gleich 3 bis 6 ist,
sowie seine Salze, seine Isomeren, wenn sie existieren, und ihre Mischungen.

2. Ein Oxazolderivat nach Anspruch 1, dadurch gekennzeichnet, daß
R und R' gleich oder verschieden sind und ein Wasserstoffatom oder einen Methylrest darstellen,
R₁ und R₂ gleich oder verschieden sind und Wasserstoffatome oder Halogenatome oder Methoxyreste darstellen,
n gleich 4 oder 5 ist,
sowie seine Salze, seine Isomeren, wenn sie existieren, und ihre Mischungen.

3. Ein Oxazolderivat nach einem der Ansprüche 1 oder 2, dadurch gekennzeichnet, daß es sich um 5-{6-[Bis-4,5-(4-Methoxy-phenyl]-2-oxazolyl]-hexyl}-tetrazol sowie seine Salze handelt.

4. Ein Oxazolderivat nach einem der Ansprüche 1 oder 2, dadurch gekennzeichnet, daß es sich um 5-{7-[Bis-4,5-(4-Methoxy-phenyl]-2-oxazolyl]-2-heptyl}-1H-tetrazol sowie seine Salze, seine Isomere und ihre Mischungen handelt.

5. Ein Oxazolderivat nach einem der Ansprüche 1 oder 2, dadurch gekennzeichnet, daß es sich um 5-{7-[Bis-4,5-(4-Methoxy-phenyl]-2-oxazolyl]-2-methyl-2-heptyl}-1H-tetrazol sowie seine Salze handelt.

6. Ein Oxazolderivat nach einem der Ansprüche 1 oder 2, dadurch gekennzeichnet, daß es sich um 5-{5-[Bis-4,5-(4-Methoxy-phenyl]-2-oxazolyl]-pentyl}-tetrazol sowie seine Salze handelt.

7. Ein Oxazolderivat nach einem der Ansprüche 1 oder 2, dadurch gekennzeichnet, daß es sich um 5-{6-[4-(4-Chlor-phenyl)-5-(4-methoxy-phenyl)]-2-oxazolyl]-hexyl}-tetrazol sowie seine Salze handelt.

8. Verfahren zur Herstellung eines Oxazolderivates nach Anspruch 1, dadurch gekennzeichnet, daß man ein Alkalinitrid oder Tri-n-Butyl-Zinn-Nitrid mit einem Nitril der allgemeinen Formel 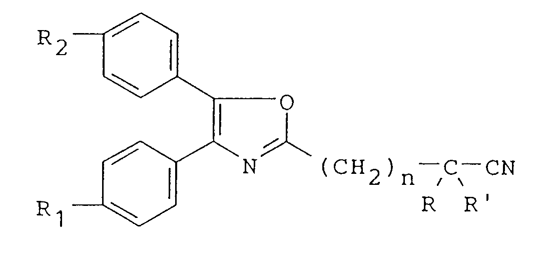 zur Reaktion bringt, in der R, R', R₁, R₂ und n wie in Anspruch 1 definiert sind, und man anschließend gegebenenfalls das erhaltene Produkt in sein Salz umwandelt.

9. Verfahren nach Anspruch 8, dadurch gekennzeichnet, daß man in Anwesenheit von Ammoniumchlorid oder dem Hydrochlorid einer organischen Stickstoffbase arbeitet, wenn man ein Alkalinitrid verwendet.

10. Verfahren nach Anspruch 8, dadurch gekennzeichnet, daß das Alkalinitrid Natriumnitrid ist.

11. Pharmazeutische Zusammensetzung, dadurch gekennzeichnet, daß sie mindestens ein Produkt nach Anspruch 1, in reinem Zustand oder in Form einer Assoziation mit einem oder mehreren, kompatiblen und pharmazeutisch akzeptablen Verdünnungsmitteln oder Zusatzstoffen enthält.

## Patentansprüche (Patentansprüche für folgende(n) Vertragsstaat(en): ES, GR)

1. Verfahren zur Herstellung eines neuen Oxazolderivates der allgemeinen Formel 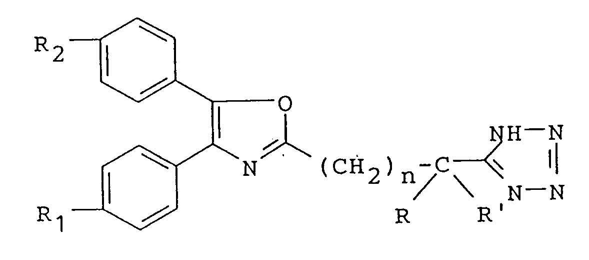 in der
R und R' gleich oder verschieden sind und ein Wasserstoffatom oder einen Alkylrest mit 1 oder 2 Kohlenstoffatomen darstellen,
R₁ und R₂ gleich oder verschieden sind und Wasserstoffatome oder Halogenatome oder Alkyloxyreste darstellen, deren Alkylteil 1 bis 4 Kohlenstoffatome in gerader oder verzweigter Kette enthält, und
n gleich 3 bis 6 ist,
sowie von seinen Salzen, seinen Isomeren, wenn sie existieren, und ihren Mischungen,
dadurch gekennzeichnet, daß man ein Alkalinitrid oder Tri-n-Butyl-Zinn-Nitrid mit einem Nitril der allgemeinen Formel 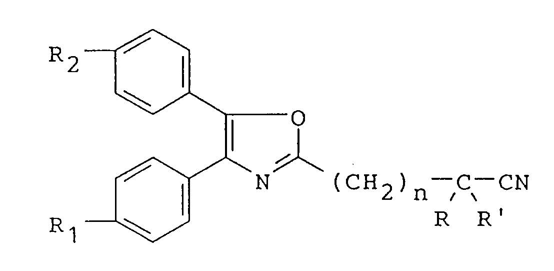 zur Reaktion bringt, in der R, R', R₁, R₂ und n wie vorstehend definiert sind, und man anschließend gegebenenfalls das erhaltene Produkt in sein Salz umwandelt.

2. Verfahren nach Anspruch 1, dadurch gekennzeichnet, daß man in Anwesenheit von Ammoniumchlorid oder dem Hydrochlorid einer organischen Stickstoffbase arbeitet, wenn man ein Alkalinitrid verwendet.

3. Verfahren nach Anspruch 1, dadurch gekennzeichnet, daß das Alkalinitrid Natriumnitrid ist.
